# FASCICULE DE BREVET EUROPEEN

(11) **EP 1 609 128 B1**
(45) Date de publication et mention de la délivrance du brevet: **13.03.2013**
(21) Numéro de dépôt: 04724020.5
(22) Date de dépôt: 29.03.2004
(51) Int. Cl.: G09F 15/00, G09F 19/00, G09F 19/22, G09F 23/00, A61L 9/14, B65D 83/16

(54) **PANNEAU D'AFFICHAGE PUBLICITAIRE AVEC DIFFUSION D'ODEURS**
WERBEANZEIGEVORRICHTUNG MIT DUFTSTOFFSPENDER
ADVERTISING DISPLAY PANEL WITH THE DIFFUSION OF SCENTS

(30) Priorité: 28.03.2003 FR 0304117
(43) Date de publication de la demande: 28.12.2005
(73) Titulaire: Prolitec Inc., Milwaukee, WI 53226 (US)
(72) Inventeur: BENALIKHOUDJA, Karim, F-34130 Mauguio (FR)
(74) Mandataire: Lawrence, John
(86) Numéro de dépôt international: PCT/EP2004/050390
(87) Numéro de publication internationale: WO 2004/086336

(56) Documents cités:
- WO-A-96/03218
- FR-A- 2 682 794
- US-A- 5 826 357
- US-A1- 2002 008 152
- US-A1- 2002 175 188
- PATENT ABSTRACTS OF JAPAN vol. 2000, no. 12, 3 janvier 2001 (2001-01-03) & JP 2000 267611 A (SANYO ELECTRIC CO LTD;TOTTORI SANYO ELECTRIC CO LTD), 29 septembre 2000 (2000-09-29)

## Description

Il est précisé que dans la présente description on entend par diffuseur d'odeur tout moyen matériel apte à produire, diffuser ou libérer une substance odorante. Le document JP 2000 26 7611 A, est consideré comme l'état de la technique le plus proche.

La présente invention a pour objet un panneau publicitaire diffuseur d'odeurs, destiné à être vu simultanément par plusieurs personnes, la nature du parfum diffusé étant par exemple en étroite relation avec le contenu du message publicitaire affiché de façon que les deux messages visuels et olfactifs renforcent mutuellement leurs effets afin d'améliorer grandement la perception du message publicitaire par le public.

La présente invention à donc pour objet un panneau publicitaire selon la revendication 1 destiné à l'affichage d'un message publicitaire destiné à être vu simultanément par plusieurs personnes et la diffusion d'une odeur dont la nature est par exemple en relation avec le contenu du message visuel affiché, ledit panneau comportant un cadre 2 constitué par l'assemblage deux montants verticaux 3 à deux traverses horizontales supérieure et inférieure 4, ledit panneau définissant un volume parallélépipédique dont l'une au moins des deux grandes faces verticales opposées comporte une fenêtre quadrangulaire d'affichage, bordée par une zone marginale périphérique de la face du panneau et dans lequel volume est disposé un ensemble d'affichage d'un message publicitaire face à la fenêtre 7, se caractérise essentiellement par au moins un diffuseur d'odeur apte à générer un flux odorant, installé dans le volume interne du panneau, ledit diffuseur d'odeur étant associé un élément diffusant d'odeur, lequel élément comporte une chambre de diffusion recevant le flux odorant, ladite chambre étant en relation de communication avec une ouverture du panneau, débouchant à l'extérieur de ce dernier pour diffuser à l'extérieur du volume interne dudit panneau le flux odorant généré par le diffuseur.

Comme on le comprend, le diffuseur d'odeur a pour objet la production d'un flux odorant sous un certain débit prédéfini et l'élément diffusant a pour objet la diffusion de ce flux odorant dans l'atmosphère externe au panneau.

Le diffuseur d'odeur et l'élément diffusant en étant totalement disposés dans le panneau publicitaire, se trouvent parfaitement protégés par ce dernier et se trouvent à l'abri de tout acte de vandalisme.

La diffusion d'une odeur dans un l'environnement immédiat d'un panneau publicitaire, placé par exemple à l'extérieur, dans des voies piétonnes et autres lieux publics, suppose que le débit du flux odorant diffusé soit suffisamment élevé pour que l'odeur soit aisément perceptible et ce très peu de temps après sa diffusion dans l'atmosphère.

À cet effet l'élément diffusant est constitué d'un corps comportant une chambre de diffusion, recevant le flux odorant possédant une première ouverture disposée en regard de l'ouverture du panneau vers l'extérieur, une seconde ouverture disposée en regard du volume interne du panneau, dans laquelle seconde ouverture est disposé un ventilateur pour pulser un flux d'air depuis le volume interne du panneau vers la ladite chambre afin que ce flux d'air se mélange au flux odorant contenu dans la chambre et que le mélange obtenu soit pulsé vers l'extérieur du panneau.

Le diffuseur d'odeur peut être constitué par un substrat imprégné d'un produit odorant volatil qui peut se présenter sous la forme d'un gel, ou d'un liquide. Le diffuseur d'odeur peut être aussi un récipient contenant des produits odorants qui peuvent se présenter sous la forme de cristaux, d'un gel, d'un liquide ou autre.

Un tel diffuseur d'odeur pourra être placé dans la chambre de sortie de l'élément diffusant.

Le diffuseur d'odeur possède une tête assurant le mélange d'un gaz porteur avec un fluide odorant contenu dans un réservoir approprié, ladite tête étant en relation de communication avec la chambre de diffusion de l'élément diffusant et possédant une sortie par laquelle est délivré le flux odorant obtenu, lequel est pulsé vers l'élément diffusant, pour être ensuite pulsé vers l'extérieur du panneau. Dans ce cas de figure, le diffuseur d'odeur est placé à l'extérieur de la chambre de sortie de l'élément diffusant et ledit élément diffusant comporte une entrée du flux odorant dans ladite chambre, connectée par une conduite à la sortie de la tête du diffuseur d'odeur.

Dans la chambre de sortie de l'élément diffusant s'opère donc le mélange entre le flux odorant délivré par la tête de mélange et le flux d'air aspiré par le ventilateur. Le mélange produit s'évacue de la chambre par passage au travers de la première ouverture et par passage au travers de l'ouverture du panneau. Cet élément diffusant, en raison du débit d'air aspiré par le ventilateur, accroît la vitesse de diffusion dans l'environnement immédiat du panneau, du mélange odorant produit. Pour compenser les effets de la dilution dans la chambre de sortie, du mélange odorant produit par la tête de mélange, la proportion de fluide odorant dans ce mélange, sera accrue, le réglage de la tête sera opéré en conséquence.

L'usage d'un élément diffusant, autorise l'emploi d'un diffuseur à faible débit de diffusion et donc à faible puissance consommée. De tels diffuseurs présentent généralement de faibles dimensions ce qui est propice à faciliter leur intégration dans le volume interne du panneau.

Selon une autre caractéristique de l'invention, le fluide odorant est un gaz odorant, le diffuseur d'odeur comportant alors un réservoir de gaz comprimé odorant, connecté par une tubulure à la tête de mélange.

Mais préférentiellement, selon une autre caractéristique de l'invention, le fluide odorant est un liquide, le diffuseur d'odeur comportant alors un réservoir de liquide odorant connecté à la tête de mélange, ladite tête assurant aussi le fractionnement du liquide en fines particules.

Dans cette dernière forme de réalisation, s'il n'est pas besoin d'un débit important en sortie du panneau, l'élément diffusant ne comportera pas de ventilateur et sera ainsi constitué d'un corps comportant une chambre de diffusion étanche recevant le flux odorant du diffuseur d'odeur, cette chambre possédant une ouverture de sortie en relation de communication avec l'ouverture du panneau et une entrée du flux odorant en relation de communication avec ledit diffuseur. Cette chambre de diffusion jouera aussi le rôle de chambre de détente de façon que les grosses particules que pourrait transporter le flux odorant se déposent sur les parois de cette dernière.

Selon une autre caractéristique de l'invention, la tête de mélange du diffuseur d'odeur comporte un corps de tête dans lequel sont formés une chambre de mélange, un premier conduit en relation de communication d'une part avec la chambre de mélange et d'autre part avec le volume interne du réservoir de fluide odorant, un deuxième conduit en relation de communication avec la chambre de mélange d'une part et avec une forme d'embout femelle d'autre part, pratiquée horizontalement dans ladite tête de manière débouchante sur la face latérale du corps de tête, ce dit deuxième conduit étant destiné à recevoir un flux d'un gaz porteur comprimé et un troisième conduit en relation de communication d'une part dans la chambre de mélange et d'autre part avec une bouche de sortie pratiquée dans la face supérieure du corps de tête, ce dit troisième conduit et ladite bouche de sortie étant destinés à conduire vers l'extérieur de la tête, le mélange gaz porteur et fluide odorant.

Selon une autre caractéristique de l'invention, le réservoir de fluide odorant et la tête de mélange sont montés dans un support fixé de manière amovible dans l'un des montants du cadre du panneau.

Selon une autre caractéristique de l'invention, le support porte un embout mâle horizontal destiné à coopérer en emboîtement de forme avec l'embout femelle de la tête de mélange, ce dit embout mâle étant connecté par une conduite à la sortie de gaz comprimé d'un compresseur que comporte le diffuseur d'odeur.

Selon une autre caractéristique de l'invention, le support porte un embout vertical, de raccordement à la bouche de sortie de la tête de mélange, ledit embout étant connecté par une conduite à l'élément diffusant.

Ces caractéristiques facilitent le retrait ou la mise en place dans le support de l'ensemble constitué par le réservoir et la tête de mélange.

Selon une autre caractéristique de l'invention, le diffuseur d'odeur est divisé en deux modules distincts.

D'autres avantages et caractéristiques de l'invention apparaîtront à la lecture de la description d'une forme préférée de réalisation, donnée à titre d'exemple non limitatif en se référant aux dessins annexés en lesquels :

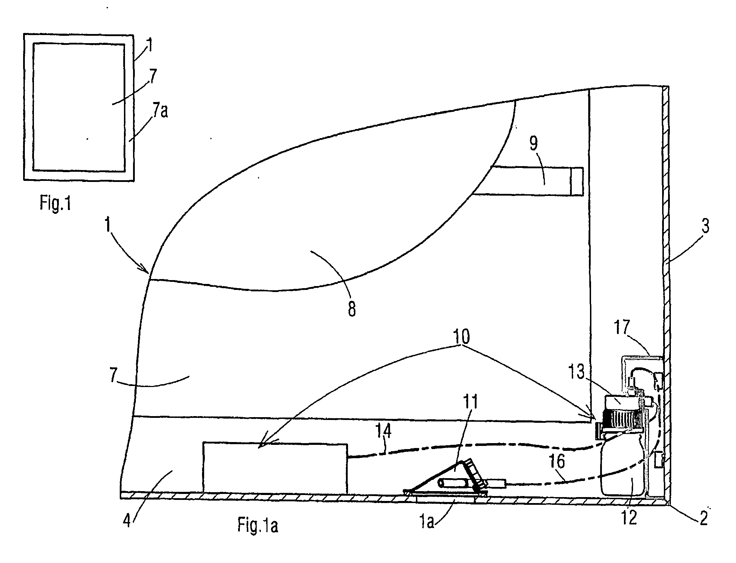

- la figure 1 est une vue de face d'un panneau publicitaire selon l'invention
- la figure 1a est une vue partielle en écorché d'un panneau selon l'invention,

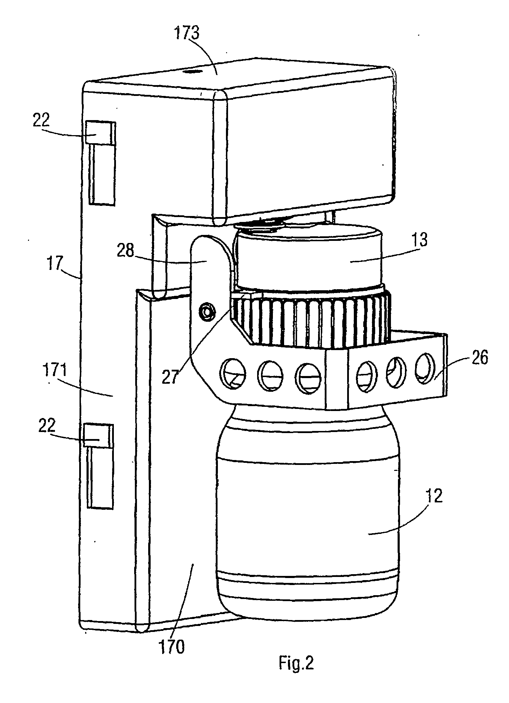

- la figure 2 est une vue en perspective de l'ensemble tête et réservoir dans son support,

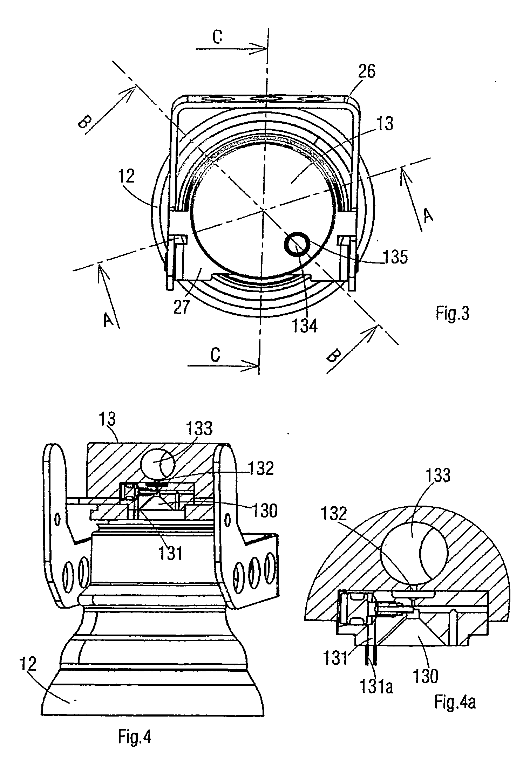

- la figure 3 est une vue dessus de la tête du diffuseur d'odeur,
- la figure 4 est une vue en coupe selon la ligne AA de la figure 3,
- la figure 4a est agrandissement de la tête représentée en figure 4,

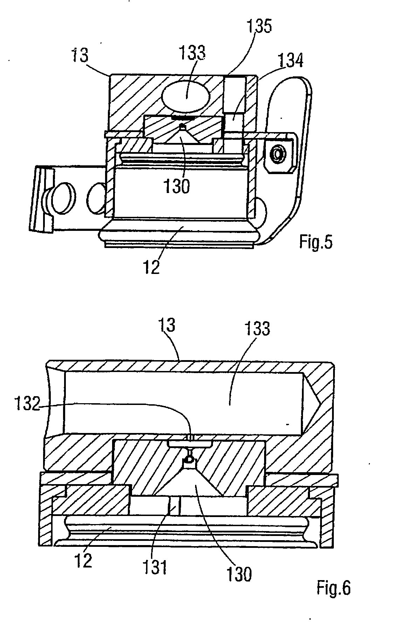

- la figure 5 est une vue en coupe selon la ligne BB de la figure 3
- la figure 6 est une vue en coupe selon la ligne CC de la figure 3

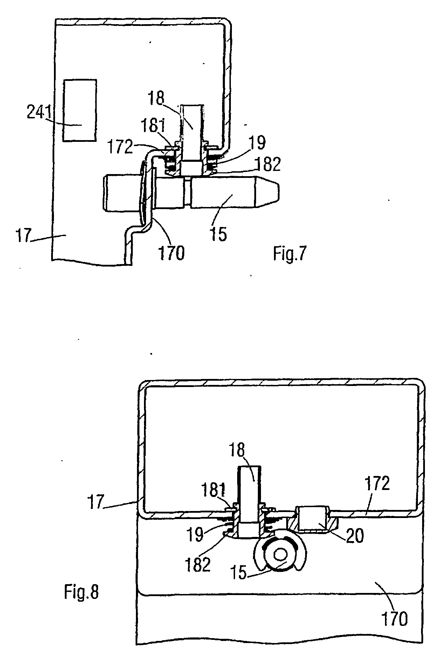

- les figures 7 et 8 sont des vues de détail du support de l'ensemble réservoir et tête du diffuseur d'odeur,

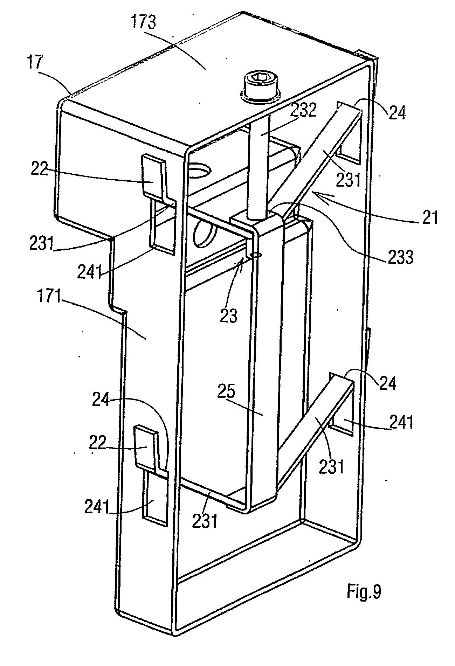

- la figure 9 est une vue du mécanisme de fixation associé au support de l'ensemble réservoir tête,

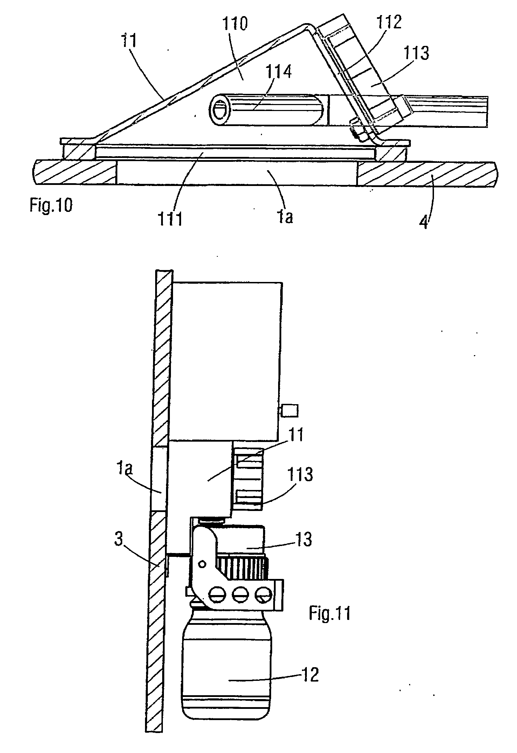

- la figure 10 est une vue en coupe de l'élément diffusant,
- la figure 11 est une vue d'un diffuseur d'odeur selon une autre forme de réalisation, dont les deux modules sont disposés côte à côte.

Tel que représenté, le panneau publicitaire 1 selon l'invention, est destiné à l'affichage d'un message publicitaire et à la diffusion d'une odeur dont la nature est en relation avec le contenu du message publicitaire affiché. Ainsi pour un produit alimentaire, le message publicitaire visuel pourra être complété par un message olfactif rappelant ce produit.

Typiquement le panneau publicitaire 1 comprend un cadre 2 constituant son ossature, formé par l'assemblage deux montants verticaux 2 à deux traverses horizontales 3 supérieure et inférieure. Le panneau 1 définit un volume parallélépipédique de faible épaisseur, comparativement à ses autres dimensions (hauteur et largeur) dont l'une au moins des deux grandes faces verticales opposées est occupée par une fenêtre quadrangulaire 7 d'affichage, bordée par une zone marginale périphérique 7a de ladite grande face du panneau. La face du panneau comportant la fenêtre 7 pourra être constituée par une paroi transparente, quadrangulaire présentant des dimensions hauteur et largeur identiques à celles du panneau. Cette paroi constituera la fenêtre 7, la zone marginale 7a sera par exemple formée par des bandes verticales et horizontales formant écran et bordant les côtés verticaux et horizontaux de ladite paroi. La fenêtre 7, comme on le comprend, est délimitée par les bandes écran. Cette paroi sera fixée au cadre du panneau par tout moyen connu.

L'autre grande face du panneau sera constituée par une paroi opaque rectangulaire fixée de manière connue au cadre du panneau.

Dans le volume interne parallélépipédique du panneau est monté un dispositif d'affichage destiné à présenter face à la fenêtre 7, une affiche publicitaire 8 amovible. Le dispositif d'affichage est en outre doté, dans le volume définit par le cadre, d'un dispositif d'éclairage constitué par un jeu de tubes éclairants 9 aptes à diffuser une lumière blanche. Ce dispositif d'éclairage assure le rétro-éclairage de l'affiche publicitaire 8.

Le dispositif d'affichage pourra être du type à affiche fixe ou bien à affiches mobiles. Dans ce cas, les affiches publicitaires seront présentées tour à tour face à l'une au moins des deux fenêtres 7.

Les montants et traverse 3, 4 du cadre pourront être constitués, sans que cela soit limitatif, par des profilés métalliques de section droite en U. Par leur intrados, ces profilés sont orientés vers le centre du panneau. En d'autres termes, la concavité que présente chaque montant ou traverse est tournée vers l'intérieur du panneau 1. Les montants et les traverses pourront être constitués aussi par des profilés tubulaires de section droite carrée ou rectangulaire.

Conformément à l'invention, le panneau publicitaire 1 est équipé d'un diffuseur d'odeur 10 associé à un élément diffusant 11 apte à diffuser à l'extérieur du panneau 1, au travers d'une ouverture 1a de ce dernier, un flux odorant généré par le diffuseur d'odeur 10 et ce sous un débit approprié.

Préférentiellement, sans que cela soit limitatif, l'ouverture 1a est formée par un perçage traversant réalisé dans une des ailes latérales ou basale de l'un des montants ou traverses. Si l'un des montants ou traverses du panneau est déjà pourvu d'une ouverture du type précité l'élément diffusant 11 sera positionné face à cette dernière, on évitera ainsi de réaliser des travaux de perçages sur ce panneau.

Sur les figures jointes, l'élément diffusant 11 est disposé dans le volume définit par la traverse inférieure 3 et l'ouverture 1a est pratiquée dans l'aile basale de cette traverse. Cette configuration présente l'avantage de rendre peut visible l'ouverture 1a, mais cette ouverture pourra être pratiquée dans l'une des ailes de l'un des deux montants 3 et l'élément diffusant logé dans ce montant. Ce sera notamment le cas lorsque les volumes internes des traverses haute et basse seront occupés par des mécanismes de défilement d'affiche.

L'élément diffusant 11 est constitué d'un corps comportant une chambre de diffusion 110 qui reçoit du diffuseur d'odeur 10 le flux odorant Cette chambre possède une première ouverture 111 disposée en relation de communication avec l'ouverture la du panneau. Ainsi cette ouverture pourra être disposée directement en regard de l'ouverture 1a du panneau, mais selon une seconde forme de réalisation l'ouverture 111 est en relation de communication étanche avec une conduite laquelle est en relation de communication avec l'ouverture 1a du panneau. Ainsi dans le cas ou les montants et traverse du panneau sont constitués chacun par un élément tubulaire la conduite assurant la communication entre l'ouverture 111 et l'ouverture 1a, sera logée en grande partie dans le montant correspondant. Dans ce cas de figure, l'ouverture 1a sera pratiquée par exemple en extrémité inférieure du montant. À distance de l'ouverture la le montant sera doté d'un perçage traversant dans lequel pourra être engagé en partie l'élément diffusant de façon à être raccordé dans montant par son ouverture 111 à la conduite précitée. On pourra prévoir aussi un élément diffusant externe au montant, dans ce cas de figure, la conduite précitée sera engagée dans le perçage traversant du montant pour être raccordée à l'ouverture 111.

Selon une forme préférée de réalisation, l'élément diffusant 11 possède une seconde ouverture 112 disposée en regard du volume interne du panneau, dans laquelle est disposé un ventilateur 113 pour pulser un flux d'air depuis le volume interne du panneau vers la chambre 110.

Le diffuseur d'odeur 10 peut être installé dans la chambre de diffusion 110 de l'élément diffusant et être constitué dans ce cas par un substrat imprégné d'un produit odorant volatil qui peut se présenter sous la forme d'un gel, ou d'un liquide ou bien, par un récipient contenant des produits odorants qui peuvent se présenter sous la forme de cristaux, d'un gel, d'un liquide ou autre.

Le diffuseur d'odeur 10 est externe à la chambre de diffusion 110 de élément diffusant et possède une tête de mélange 13 apte à assurer le mélange d'un gaz porteur avec un fluide odorant contenu à l'état concentré dans un réservoir approprié 12. Cette tête de mélange 13 comporte une bouche de sortie 135 par laquelle est délivré le flux odorant obtenu, lequel est pulsé vers l'élément diffusant 11, pour être ensuite pulsé vers l'extérieur du panneau 1.

Dans le cadre de cette forme de réalisation du diffuseur d'odeur, l'élément diffusant 11 comporte une entrée 114 du flux odorant dans la chambre 110, en relation de communication avec la sortie 135 de la tête de mélange 13. Cette entrée 114 est matérialisée par une tubulure pénétrant dans la chambre de diffusion 110.

Préférentiellement, les première 111 et seconde 112 ouvertures de la chambre de diffusion 110 de l'élément diffusant 11 sont en regard l'une de l'autre, et l'entée 114 du flux odorant dans ladite chambre est oblique ou perpendiculaire par rapport à un axe géométrique sécant aux première et seconde ouverture, ladite entrée étant orientée vers la première ouverture.

Cette disposition créée un effet Venturi dans la chambre de diffusion facilitant l'introduction dans ladite chambre du mélange odorant produit par la tête de mélange et de fractionnement 13. En d'autres termes ce mélange odorant se trouve aspiré dans la chambre de diffusion.

L'élément diffusant 11 autour de sa première ouverture 111 pourra être équipé d'un joint d'étanchéité souple par lequel il sera fixé à la traverse inférieure 4.

Le diffuseur d'odeur 10 et l'élément diffusant 11 sont latéraux à la fenêtre 7 et sont masqués par la zone marginale 7a périphérique de la fenêtre 7. Ainsi on évite la formation d'ombres sur l'affiche publicitaire 8, ombres qui pourrait affecter l'esthétique de l'affiche d'une part et brouiller ou altérer le message publicitaire d'autre part. Le diffuseur d'odeur 10 et l'élément diffusant 11 pourront être logés en totalité ou en partie dans le volume de l'un au moins montant ou traverse du cadre.

Le diffuseur d'odeur 10 pourra être divisé en plusieurs modules fonctionnels pour une meilleure intégration en arrière de la zone marginale périphérique 7a de la fenêtre 7, par exemple dans le volume de l'un montant 3 ou traverse 4 du cadre 2. Ces divers modules pourront être fixés par collage ou par des systèmes de fixation amovible par adhérence. On évite ainsi tout travaux d'usinage sur les montants et traverses.

Selon une forme préférée de réalisation, le diffuseur d'odeur 10 est divisé essentiellement en deux modules fonctionnels distincts pouvant être disposés à distance l'un de l'autre. Par exemple le premier de ces modules sera disposé dans le volume définit par la traverse inférieure 4, tandis que le second sera disposé dans le volume définit par un des deux montants 3.

Le premier module est constitué d'une unité de contrôle et de commande du fonctionnement du diffuseur d'odeur et d'un compresseur actionné par un moteur électrique de faible puissance. Cette unité et le moteur électrique du compresseur seront connectés électriquement à l'alimentation électrique du dispositif d'éclairage. Le compresseur est destiné à délivrer un flux de gaz porteur sur sa tubulure de sortie, cette tubulure étant connectée par une conduite appropriée 14 à la tête de mélange 13. Préférentiellement le gaz porteur est de l'air comprimé.

L'unité de contrôle et de commande ainsi que le compresseur d'air seront logés dans un même caisson de forme parallélépipédique de dimensions adaptées à celles du volume interne de la traverse ou montant qui le reçoit afin d'être totalement logé dans le volume de ce montant ou traverse. Préférentiellement ce caisson sera fixé par collage à cette traverse.

Le second module est constitué par le réservoir 12 et la tête de mélange 13. La tête de mélange 13 comporte un corps de tête dans lequel sont formés une chambre de mélange 130, un premier conduit 131 en relation de communication d'une part avec la chambre de mélange 130 et d'autre part avec le volume interne du réservoir 12, un deuxième conduit 132 en relation de communication avec la chambre de mélange 130 d'une part et avec une forme d'embout femelle 133 d'autre part pratiquée horizontalement dans ladite tête de manière débouchante sur la face latérale du corps de tête, ce dit deuxième conduit 132 étant destiné à recevoir un flux d'un gaz comprimé porteur et un troisième conduit 134 en relation de communication d'une part dans la chambre de mélange 130 et d'autre part avec une bouche de sortie 135 pratiquée dans la face supérieure du corps de tête, ce dit troisième conduit 134 et ladite bouche de sortie 135 étant destinés à conduire vers l'extérieur de la tête 13, le mélange gaz porteur et fluide odorant. Préférentiellement, sans que cela soit limitatif, le fluide odorant est aspiré dans la chambre de mélange 130 par effet Venturi, cet effet étant créé par le flux de gaz porteur au travers de la chambre de mélange.

Le premier conduit 131 est préférentiellement connecté de manière étanche à une conduite 131a plongeant dans le réservoir 12. La forme d'embout femelle 133 reçoit de manière amovible un embout mâle 15. Cet embout mâle est monté en extrémité de la conduite 14, cette conduite étant raccordée à la tubulure de sortie du compresseur. La bouche de sortie 135 est connectée par une conduite 16 à l'élément diffusant 11 plus précisément à l'entrée 114 de l'élément diffusant 11.

Le fluide contenu dans le réservoir peut être un gaz odorant mais, selon la forme préférée de réalisation, le fluide odorant est un liquide et la tête de mélange 13 dans la chambre de mélange 130 assure aussi le fractionnement du liquide en fines particules. Préférentiellement la tête 13 de mélange et de fractionnement, produit un nébulisat et, dans ce cas, le diffuseur d'odeur est un nébuliseur. Le mélange délivré par l'élément diffusant sera par voie de conséquence un nébulisat. Il faut rappeler qu'un nébulisat est constitué de particules de taille inférieure au micron. L'intérêt de produire un nébulisat est multiple. D'une part, le flux délivré par l'élément diffusant est visuellement indiscernable, mais aussi pour une quantité de liquide égale, un nébulisat contient un nombre de particules bien plus élevé qu'une autre forme de production d'un nuage odorant, atomisat ou autre, ce qui procure l'obtention d'un effet olfactif renforcé. Un autre intérêt de produire un nébulisat, c'est-à-dire un nuage composé de très fines particules réside dans le fait, que contrairement à un flux comportant de grosses particules, le risque de pollution par dépôt, tant à l'intérieur du panneau qu'à l'extérieur devient inexistant ceci en raison de la charge électrostatique des particules et de leur évaporation rapide dans l'atmosphère de par leur vitesse de sortie. S'il existe un risque de présence de grosses particules dans le flux odorant délivré par la tête 13 du diffuseur d'odeur 10, l'élément diffusant 11 ne comporte pas de ventilateur 113 et est constitué d'un corps comportant une chambre de diffusion étanche recevant le flux odorant du diffuseur d'odeur, cette chambre possédant une ouverture de sortie en relation de communication avec l'ouverture 1a du panneau et une entrée du flux odorant en relation de communication avec ledit diffuseur. Cette chambre de diffusion remplit un rôle de chambre de détente de façon que les grosses particules que pourrait transporter le flux odorant se déposent sur les parois de cette dernière.

Selon la forme préférée de réalisation, la tête 13 est fixée sur le buvant du réservoir 12, de manière démontable ou indémontable.

Avantageusement, le réservoir 12 et la tête 13 sont montés dans un support 17 fixé de manière amovible dans l'un des montants du panneau. Ce support assure le maintien à la verticale du réservoir et de la tête.
Ce support 17 comme on peut le voir, comprend une aile verticale plane frontale 170 bordée par deux ailes verticales latérales 171 se développant de manière parallèle l'une à l'autre en arrière de l'aile frontale 170. Le réservoir 12 et la tête de mélange 13 sont montés en avant de l'aile frontale face à cette dernière. L'aile frontale 170 est équipée d'un perçage traversant dans lequel est monté en fixation l'embout mâle 15, ce dernier se développant horizontalement de part et d'autre de ladite paroi. En arrière de la paroi, l'embout mâle 15 est raccordé à la conduite 14. La tête 13 par l'embout femelle 133 est engagée sur l'embout mâle 14.

Le support 17 est de plus équipé d'un embout 18 vertical de raccordement à la bouche de sortie 135 de la tête 13, cet embout 18 étant connecté à la conduite 16. Préférentiellement, cet embout 18 se raccorde à la bouche par simple emboîtement de forme ce qui facilite la mise en place ou le retrait de l'ensemble tête 13 et réservoir 12 du support 17.

Préférentiellement, l'embout de raccordement 18 est porté de manière flottante par le support 17 et est appliqué contre la bouche de sortie 135 de la tête de mélange 13 par un organe élastique 19 monté en compression entre le support 17 et ledit embout 18. De part l'action de poussée exercée par l'organe élastique 19, l'embout 18 demeure en place dans la bouche de sortie 135 et ce sans risque de fuite de nébulisat. Pour renforcer encore l'étanchéité à ce niveau, un joint torique, élastiquement déformable pourra être disposé dans un lamage de la bouche de sortie 135 et l'embout de raccordement 18, par l'action du ressort 19 sera appliqué contre ce joint élastique. Le fait que l'embout de raccordement 18 soit porté de manière flottante par le support 17 permet le rattrapage des défauts de positionnement de bouche de sortie 135 la tête 13 par rapport au dit embout 18.

Selon la forme préférée de réalisation, l'embout de raccordement 18, par une section cylindrique de son corps, limitée par un épaulement supérieur 181 et un épaulement inférieur 182, est engagé avec jeu, dans un perçage pratiqué dans une aile horizontale 172 du support 17, le jeu fonctionnel conférant audit embout 18 une latitude déplacement axial et de pivotement limités, la tête de mélange 13 et le réservoir 12 étant positionnés sous ladite aile 172, cette dernière s'étendant vers l'avant de l'aile frontale 170.

L'organe élastique 19 est un ressort à spires disposé autour de la section cylindrique du corps de l'embout 18, et monté en compression entre l'aile horizontale 172 du support 17 et l'épaulement inférieur 182 de l'embout 18.

Préférentiellement est prévue une butée 20 fixée à l'aile horizontale 172 du support 17, formant saillie vers le bas, sous laquelle vient la face supérieure de la tête 13, ladite butée 20 étant destinée à venir limiter le mouvement de pivotement, autour de l'embout mâle 15, de la tête 13 et du réservoir 12, dans un sens correspondant à l'éloignement angulaire de la bouche 135 par rapport à l'embout 18, le mouvement de pivotement opposé étant contré par l'embout 18. Cette disposition assure un calage angulaire de l'ensemble réservoir et tête dans le support, tout en permettant cependant un mouvement de pivotement de fable amplitude dans un sens ou dans l'autre limité à quelques degrés, pour faciliter la mise en place ou le retrait dudit ensemble tête et réservoir de son support 17.

Le support 17 est positionné dans le volume définit par le montant correspondant de telle manière que ses ailes latérales 171 soient parallèles aux ailes latérales de ce montant, et orientées vers l'aile basale de ce dernier. L'ensemble réservoir 12 et tête 13 se trouve tourné vers l'ouverture de ce montant pour être facilement accessible.

Dans le support 17, au-dessus du second module pourra être monté le premier module ainsi que l'élément diffusant 11. On forme ainsi un ensemble d'un seul tenant qui sera disposé verticalement dans l'une des traverses du cadre.

Le support 17 pourra être pourvu d'un système de fixation amovible 21, par adhérence, dans le montant 3 correspondant du cadre 2 du panneau 1. Ce système de fixation coopère avec l'une au moins des ailes latérales du montant et est constitué par un tampon de serrage 22 monté en extrémité d'un dispositif de manoeuvre 23. Par action sur ce dispositif, le tampon de serrage est amené en pression contre l'une des ailes latérales pour réaliser l'immobilisation ou en est éloigné. Ce dispositif de manoeuvre 23 comprend au moins un bras 231 élastiquement flexible en extrémité distale duquel est disposé le tampon de serrage 22, et un mécanisme du type vis 232 et écrou 233 dont l'écrou 233 est fixé de manière rigide en extrémité proximale du bras 232 et dont la vis 232 est disposée verticalement, est engagée dans un perçage traversant, pratiquée dans une aile horizontale supérieure 173 du support, ladite vis 232 étant bloquée en translation et libre en rotation par rapport audit support 17 et ledit bras 231 se développant de manière oblique par rapport à la vis 232 et venant en appui contre une butée radiale fixe 24, de façon que par manoeuvre de la vis 232 l'oblicité du bras soit modifiée, par déformation élastique dudit bras, et que ledit bras pivote et glisse sur la butée 24 ce qui se traduit par un mouvement d'éloignement ou de rapprochement du tampon 22 par rapport à la vis. Préférentiellement le tampon 22 sera formé par un repliement du bras 231.

Le bras 231 est engagé par sa zone distale dans une ouverture 241 pratiquée dans une aile verticale latérale 171 du support 17, l'une des lèvres supérieure ou inférieure de la dite ouverture selon que l'extrémité proximale du bras 231 est plus basse ou plus haute que l'extrémité distale, constituant la butée radiale fixe 24.

Selon une forme préférée de réalisation, le dispositif de manoeuvre 23 comprend deux bras opposés 231 comportant chacun un tampon de serrage 22, et engagés dans deux ouvertures 241 pratiqués dans les ailes latérales verticales 171 du support 17. Le système de fixation 21 coopère alors avec les deux ailes latérales du montant.

De préférence pour améliorer la qualité de la fixation, le dispositif de manoeuvre 23 comprend deux paires de bras opposés 231 à savoir une paire de bras supérieure et une paire de bras inférieure, mécaniquement jointes l'une à l'autre par une barre de liaison 25 et de transmission d'effort. Seront ménagées dans les ailes latérales 171 une paire d'ouvertures supérieure et une paire d'ouverture inférieure 241. La fixation du support 17 dans le montant correspondant 3 sera réalisée selon quatre points supérieur et inférieur.

Pour faciliter le retrait de l'ensemble tête 13 et réservoir 12, sera adjoint à cet ensemble une poignée de manoeuvre 26. Cette poignée de manoeuvre est articulée à une platine 27 insérée entre la tête 13 et le réservoir 12 et comporte au-delà de ses articulations deux formes de bras 28 aptes à venir agir en poussée contre la paroi frontale 170 du support 17 lors de sa manoeuvre en basculement vers le haut.

## Revendications

1. Panneau publicitaire (1) destiné à l'affichage d'un message publicitaire destiné à être vu simultanément par plusieurs personnes et la diffusion d'une odeur dont la nature est par exemple en relation avec le contenu du message visuel affiché, ledit panneau (1) comportant un cadre (2) constitué par l'assemblage deux montants verticaux (3) à deux traverses horizontales supérieure et inférieure (4), ledit panneau définissant un volume parallélépipédique dont l'une au moins des deux grandes faces verticales opposées comporte une fenêtre quadrangulaire d'affichage (7) bordée par une zone marginale périphérique (7a) de la face du panneau et dans lequel volume est déposé un ensemble d'affichage d'un message publicitaire face à la fenêtre (7), par au moins un diffuseur d'odeur (10) apte à générer un flux odorant, installé dans le volume interne du panneau (1) et associé un élément diffusant d'odeur (11) dans le volume interne du panneau lequel élément (11) comporte une chambre de diffusion (110) recevant le flux odorant, ladite chambre (110) étant en relation de communication avec une ouverture (1a) du panneau débouchant à l'extérieur de ce dernier pour diffuser à l'extérieur du volume interne dudit panneau un flux odorant généré par le diffuseur **caractérisé en ce que** le diffuseur d'odeur (10) possède une tête (13) assurant le mélange d'un gaz porteur avec un fluide odorant contenu dans un réservoir approprié (12), ladite tête (13) étant en relation de communication avec la chambre de diffusion de l'élément diffusant et possédant une sortie par laquelle est délivré le flux odorant obtenu, lequel est pulsé vers l'élément diffusant (11), pour être ensuite pulsa vers l'extérieur du panneau.

2. Panneau publicitaire selon l'une quelconque des revendications précédente, **caractérisé en ce que** l'élément diffusant (11) est constitué d'un corps comportant une chambre de diffusion (110) recevant le flux odorant, ladite chambre possédant une première ouverture (111) disposée en regard de l'ouverture (1a) du panneau, une seconde ouverture (112) disposée en regard du volume interne du panneau, dans laquelle ouverture (112) est disposé un ventilateur (113) pour pulser un flux d'air depuis le volume interne du panneau vers la ladite chambre (110) afin que ce flux d'air se mélange au flux odorant contenu dans la chambre et que le mélange obtenu soit pulsé vers l'extérieur du panneau.

3. Panneau publicitaire selon les revendications 1 et 2 prises ensembles, **caractérisé en ce que** l'élément diffusant (11) comporte une entrée (114) du flux odorant dans ladite chambre, connectée par une conduite (16) à la sortie de la tête (13) du diffuseur d'odeur.

4. Panneau publicitaire selon la revendication 3, **caractérise en ce que** les première (111) et seconde (112) ouvertures de la chambre de diffusion (110) de l'élément diffusant (11) sont en regard l'une de l'autre, et que l'entrée (114) du flux odorant dans ladite chambre (110) est oblique ou perpendiculaire par rapport à un axe géométrique sécant aux première (111) et seconde (112) ouvertures, ladite entrée étant orientée vers la première ouverture,

5. Panneau publicitaire selon l'une quelconque des revendications précédentes, **caractérisé en ce que** le fluide odorant est un gaz odorant, le diffuseur d'odeur (10) comportant alors un réservoir de gaz comprimé odorant, connecté par une tubulure à la tête de mélange (18).

6. Panneau publicitaire selon l'une quelconque des revendications précédentes, **caractérisé en ce que** le fluide odorant est un liquide, le diffuseur d'odeur (10) comportant alors un réservoir de liquide odorant connecté à la tête de mélange (13), ladite tête assurant aussi le fractionnement du liquide en fines particules.

7. Panneau publicitaire selon la revendication 6, **caractérisé en ce que** le mélange diffusé est un nébulisat et que le diffuseur d'odeur (10) est un nébuliseur.

8. Panneau publicitaire selon l'une quelconque des revendications précédentes, **caractérisé en ce que** le diffuseur d'odeur (10) est divisé en deux modules distincts dont le premier est constitué par une unité de contrôle et de commande du fonctionnement du diffuseur d'odeur et d'un compresseur actionné par un moteur électrique et dont le second est constitué par le réservoir (12) et la tête (13).

9. Panneau publicitaire selon l'une quelconque des revendications précédentes, **caractérisé en ce que** la tête (13) est fixée sur le buvant du réservoir (12).

10. Panneau publicitaire selon l'une quelconque des revendications précédentes, **caractérisé en ce que** le réservoir (12) et la tête (13) sont montés dans un support (17) fixé de manière amovible dans l'un des montents (3) du cadre (2) du panneau (1).

11. Panneau publicitaire selon la revendication 10, **caractérisé en ce que** le support (17) porte un embout mâle horizontal (15) destiné à coopérer en emboîtement de forme avec l'embout femelle (133) de la tête de mélange (13), ce dit embout mâle (15) étant connecté par une conduite (14) à la sortie de gaz comprimé d'un compresseur que comporte le diffuseur d'odeur (10).

12. Panneau publicitaire selon la revendication 10 ou la revendication 11, **caractérisé en ce que** le support (17) porte un embout vertical (18), de raccordement à la bouche de sortie (135) de la tête de mélange (13), ledit embout (18) étant connecté par une conduite (16) à l'élément diffusant (11).

13. Panneau publicitaire selon la revendication 12, **caractérisé en ce que** l'embout de raccordement (18) est porté de manière flottante par le support (17) et est appliqué contre la bouche de sortie (135) de la tête de mélange (13) par un organe élastique (19).

14. Panneau publicitaire selon la revendication 13 **caractérisé en ce que** l'embout de raccordement (18), par une section cylindrique de son corps, limitée par un épaulement supérieur (181) et un épaulement inférieur (182), est engagé avec jeu, dans un perçage pratiqué dans une aile horizontale (172) du support (17), le jeu fonctionnel conférant audit embout (18) une latitude déplacement axial et de pivotement limités, la tête de mélange (13) et le réservoir (12) étant positionnés sous ladite aile (172).

15. Panneau publicitaire selon la revendication 14, **caractérisé en ce que** l'organe élastique (19) est un ressort à spires disposé autour de la section cylindrique du corps de l'embout (18), et monté en compression entre l'aile horizontale (172) du support (17) et l'épaulement inférieur (182) de l'embout (16).

16. Panneau publicitaire selon l'une quelconque des revendications 13 à 15, **caractérisé par** une butée (20) fixée à l'aile horizontale (172) du support (17), formant saillie vers le bas, sous laquelle vient la face supérieure de la tête (13), ladite butée (20) étant notamment destinée à venir limiter le mouvement de pivotement, autour de l'embout mâle (15), de la tête (13) et du réservoir (12), dans un sens correspondant à l'éloignement angulaire de la bouche (135) par rapport à l'embout (18), le mouvement de pivotement opposé étant contré par l'embout (18).

17. Panneau publicitaire selon l'une quelconque des revendications 10 à 16, **caractérisé en ce que** le support (17) est pourvu d'un système (21) de fixation amovible, par adhérence, dans le montant (3) correspondant du cadre (2) du panneau (1), ce système de fixation coopérant avec l'une au moins des alles latérales de co montant.

18. Panneau publicitaire selon la revendication 17, **caractérisé en ce que** le système de fixation amovible (21) par adhérence, est constitué par au moins un tampon de serrage (22), monté en extrémité d'un dispositif de manoeuvre (23).

19. Panneau publicitaire selon la revendication 18, **caractérisé en ce que** le dispositif de manoeuvre comprend au moins un bras (231) élastiquement flexible en extrémité distale duquel est disposé le tampon de serrage (22), et un mécanisme du type vis (232) et écrou (233) dont l'écrou (233) est fixé de manière rigide en extrémité proximale du bras (232) et dont la vis (232) est disposée verticalement, est engagée dans un perçage traversant pratiquée dans une aile horizontale supérieure (173) du support, ladite vis (232) étant bloquée en translation et libre en rotation par rapport audit support (17) et ledit bras (231) se développant de manière oblique par rapport à la vis (232) et venant en appui contre une butée radiale fixe (24), de façon que par manoeuvre de la vis (232) l'oblicité du bras soit modifiée et que ledit bras pivote et glisse sur la butée (24) ce qui se traduit par un mouvement d'éloignement ou de rapprochement du tampon (22) par rapport à la vis (232).

20. Panneau publicitaire selon la revendication 19, **caractérisé en ce que** le bras (231) est engagé par sa zone distale dans une ouverture (241) pratiquée dans une aile verticale latérale (171) du support (17), l'une des lèvres supérieure ou inférieure de ladite ouverture selon que l'extrémité proximale du bras (231) est plus basse ou plus haute que l'extrémité distale, constituant la butée radiale fixe (24).

21. Panneau publicitaire selon la revendication 20, **caractérisé en ce que** le dispositif de manoeuvre (23) comprend deux bras opposés (231) comportant chacun un tampon de serrage (22), et engagés dans deux ouvertures (241) pratiquées dans les ailes latérales verticales (171) du support (17).

22. Panneau publicitaire selon la revendication 21, **caractérisé en ce que** le dispositif de manoeuvre (23) comprend deux paires de bras opposés (231) à savoir une paire de bras supérieure et une paire de bras inférieure, mécaniquement Jointes l'une à l'autre par une barre de liaison (25) et de transmission d'effort.

23. Panneau publicitaire selon la revendication 2, **caractérisé en ce que** le diffuseur d'odeur (10) est installé dans la chambre de diffusion (110) de l'élément diffusant (11).

24. Panneau publicitaire selon la revendication précédente, **caractérisé en ce que** le diffuseur d'odeur (10) est constitué par un substrat imprégné d'un produit odorant volatil qui peut se présenter sous la forme d'un gel, ou d'un liquide.

25. Panneau publicitaire selon la revendication 23, **caractérisé en ce que** le diffuseur d'odeur (10) est un récipient contenant des produits odorants qui peuvent se présenter sous la forme de cristaux, d'un gel, d'un liquide ou autre.

26. Panneau publicitaire selon l'une quelconque des revendications précédentes, **caractérisé en ce que** le diffuseur d'odeur (10) et l'élément diffusant (11) sont latéraux à la fenêtre (7) et sont masqués par la zone marginale périphérique (7a) de ladite fenêtre.

## Claims

1. Advertising hoarding (1) intended for the display of an advertising message destined to be seen simultaneously by many people and for the diffusion of a scent whose nature, for example, relates to the contents of the visual message displayed, said hoarding (1) including a frame (2) constituted through the assembly of two vertical uprights (3) with two horizontal crosspieces (4), upper and lower, said hoarding defining a parallelepipedic volume, at least one of whose two large opposed vertical faces includes a quadrangular display window (7) bordered by a peripheral marginal area (7a) of the hoarding face and in which volume an advertising message display assembly is arranged facing the window (7) wherein at least one scent diffuser (10) capable of generating a scented flow, installed within the internal volume of the hoarding (1) and an associated scent diffuser element (11) within the internal volume of the hoarding, said element (11) including a diffusion chamber (110) receiving the scented flow, said chamber (110) being in a communicating relationship with an opening (1a) in the hoarding emerging outside the latter so as to spray a scented flow generated by the diffuser outside the internal volume of the said hoarding, **characterised in that** the scent diffuser (10) possesses a head (13) providing a mixture of a carrier gas with a scented flow contained in an appropriate tank (12), said head (13) being in a communicating relationship with the diffusion chamber of the diffusing element and possessing an outlet through which the scented flow obtained is delivered, this being propelled towards the diffusing element (11) in order to then be propelled towards the exterior of the hoarding.

2. Advertising hoarding according to any of the previous claims, **characterised in that** the diffusing element (11) is constituted of a body including a diffusion chamber (110) receiving the scented flow, said chamber possessing a first opening (111) arranged facing the opening (1a) of the hoarding, a second opening (112) arranged facing the internal volume of the hoarding, in which opening (112) a ventilator (113) is arranged in order to propel a flow of air from the internal volume of the hoarding towards the said chamber (110) so that this air flow mixes with the scented flow contained in the chamber and the mixture obtained is propelled towards the exterior of the hoarding.

3. Advertising hoarding according to claims 1 and 2 taken together, **characterised in that** the diffusing element (11) includes an inlet (114) for the scented flow in the said chamber, connected via a pipe (16) to the outlet from the head (13) of the scent diffuser.

4. Advertising hoarding according to claim 3, **characterised in that** the first (111) and second (112) openings of the diffusion chamber (110) of the diffusing element (11) are opposite another, and that the inlet (114) of the scented flow into the said chamber (11) is oblique or perpendicular in relation to a geometrical axis intersecting the first (111) and second (112) openings, said inlet being oriented towards the first opening.

5. Advertising hoarding according to any of the previous claims, **characterised in that** the scented fluid is a scented gas, the scent diffuser (10) therefore including a tank of scented compressed gas, connected via piping to the mixing head (13).

6. Advertising hoarding according to any of the previous claims, **characterised in that** the scented fluid is a liquid, the scent diffuser (10) therefore including a tank of scented liquid connected to the mixing head (13), said head also ensuring the division of the liquid into fine particles.

7. Advertising hoarding according to claim 6, **characterised in that** the mixture sprayed is a nebulisate and that the scent diffuser (10) is a nebuliser.

8. Advertising hoarding according to any of the previous claims, **characterised in that** the scent diffuser (10) is divided into two separate modules, the first of which includes a unit for the functional command and control of the scent diffuser and a compressor worked by an electric motor and the second of which includes the tank (12) and the head (13).

9. Advertising hoarding according to any of the previous claims **characterised in that** the head (13) is fixed onto the lip of the tank (12).

10. Advertising hoarding according to any of the previous claims, **characterised in that** the tank (12) and the head (13) are mounted on a bracket (17) detachably fixed within one of the uprights (3) of the frame (2) of the hoarding (1).

11. Advertising hoarding according to claim 10, **characterised in that** the bracket (17) holds a horizontal male nozzle (15) intended to cooperate in an interlocking form with the female nozzle (133) of the mixing head (13), this said male nozzle (15) being connected by a pipe (14) to the compressed gas outlet of a compressor included in the scent diffuser (10).

12. Advertising hoarding according to claim 10 or claim 11, **characterised in that** the bracket (17) holds a vertical nozzle (18) for connection to the mouth of the outlet (135) of the mixing head (13), said nozzle (18) being connected by a pipe (16) to the diffusing element (11).

13. Advertising hoarding according to claim 12, **characterised in that** the connecting nozzle (18) is floatingly held by the bracket (17) and is applied against the mouth of the outlet (135) of the mixing head (13) by an elastic organ (19).

14. Advertising hoarding according to claim 13, **characterised in that** the connecting nozzle (18), through a cylindrical section of its body limited by an upper shoulder (181) and a lower shoulder (182), is loosely engaged in a bore hole made in a horizontal wing (172) of the bracket (17), the functional looseness conferring upon the said nozzle (18) a latitude for axial displacement and for limited pivoting, the mixing head (13) and the tank (12) being positioned under the said wing (172).

15. Advertising hoarding according to claim 14, **characterised in that** the elastic organ (19) is a spiral spring arranged around the cylindrical section of the body of the nozzle (18) and mounted in compression between the horizontal wing (172) of the bracket (17) and the lower shoulder (182) of the nozzle (18).

16. Advertising hoarding according to any of claims 13 to 15, **characterised by** a stop part (20) fixed to the horizontal wing (172) of the bracket (17), forming a downward projection, under which the upper face of the head (13) arrives, said stop part (20) being, among other things, intended to act to limit the pivoting movement around the male nozzle (15) of the head (13) and the tank (12) in a direction corresponding to the angular removal of the mouth (135) in relation to the nozzle (18), the opposite pivoting movement being countered by the nozzle (18).

17. Advertising hoarding according to any of claims 10 to 16 **characterised in that** the bracket (17) is equipped with a system (21) for detachable attachment, through adherence, in the corresponding upright (3) of the frame (2) of the panel (1), this attachment system cooperating with at least one of the lateral wings of this upright.

18. Advertising hoarding according to claim 17, **characterised in that** the detachable attachment system (21) through adherence includes at least one clamping plug (22), mounted at the end of a handling device (23).

19. Advertising hoarding according to claim 18, **characterised in that** the handling device includes at least one elastically flexible arm (231) at the distal end of which a clamping plug (22) is arranged, and a screw (232) and nut (233) type mechanism, whose nut (233) is attached rigidly at the proximal end of the arm (232) and whose screw (232) is arranged vertically, is engaged in a through-bore made in an upper horizontal wing (173) of the bracket, said screw (232) being blocked in translation and free in rotation in relation to the said bracket (17) and the said arm (231) extending obliquely in relation to the screw (232) and coming to rest against a fixed radial stop part (24), so that by handling the screw (232) the oblique nature of the arm is changed and the said arm pivots and slides on the stop part (24), which causes the plug (22) to move away from or closer to the screw (232).

20. Advertising hoarding according to claim 19, **characterised in that** the arm (231) is engaged by its distal zone in an opening (241) made in a vertical side wing (171) of the bracket (17), the fixed radial stop part (24) being constituted of one of the upper or lower lips of the said opening, depending on whether the proximal end of the arm (231) is lower or higher than the distal end.

21. Advertising hoarding according to claim 20, **characterised in that** the handling device (23) includes two opposing arms (231), each including a clamping plug (22) and engaged in two openings (241) made in the vertical side wings (171) of the bracket (17).

22. Advertising hoarding according to claim 21, **characterised in that** the handling device (23) includes two pairs of opposing arms (231), namely a pair of upper arms and a pair of lower arms, mechanically joined to one another via a connecting rod (25) that transmits stress.

23. Advertising hoarding according to claim 2, **characterised in that** the scent diffuser (10) is installed in the diffusion chamber (110) of the diffusing element (11).

24. Advertising hoarding according to the previous claim **characterised in that** the scent diffuser (10) includes a substrate impregnated with a volatile scented product that may take the form of a gel or a liquid.

25. Advertising hoarding according to claim 23, **characterised in that** the scent diffuser (10) is a receptacle containing scented products that may take the form of crystals, a gel, a liquid or some other form.

26. Advertising hoarding according to any of the previous claims **characterised in that** the scent diffuser (10) and the diffusing element (11) are lateral to the window (7) and are masked by the peripheral marginal area (7a) of the said window.

## Patentansprüche

1. Werbeanzeigevorrichtung (1) zur Anzeige einer Werbebotschaft, die von vielen Menschen gleichzeitig gesehen werden soll, und zum Verströmen eines Duftstoffs, dessen Beschaffenheit sich zum Beispiel auf den Inhalt der gezeigten visuellen Botschaft bezieht, wobei diese Anzeigevorrichtung (1) einen Rahmen (2) aufweist, der sich aus der Anordnung von zwei vertikalen Seitenteilen (3) mit zwei horizontalen Stegen (4) - einer davon unten und einer oben - zusammensetzt, und wobei die Anzeigevorrichtung ein quaderförmiges Volumen definiert, von dem mindestens eine der beiden großen vertikalen Flächen ein viereckiges Bildschirmfenster (7) umfasst, welches von einem peripheren Randbereich (7a) der Anzeigevorrichtung umgeben ist, und wobei sich in diesem Volumen eine Werbebotschaftsanzeigeeinheit gegenüber dem Bildschirmfenster (7) befindet, worin mindestens ein Duftdiffusor (10), der zur Erzeugung eines Duftstroms in der Lage ist, sowie ein damit verbundenes Duftdiffusorelement (11) innerhalb des Innenvolumens der Anzeigevorrichtung (1) installiert ist, wobei dieses Element (11) eine Diffusionskammer (110) beinhaltet, die den Duftstrom aufnimmt und im Austausch mit einer Öffnung (1a) in der Anzeigevorrichtung, aus der diese Öffnung austritt, steht, damit ein Duftstrom, der vom Diffusor außerhalb des Innenvolumens der genannten Anzeigevorrichtung erzeugt wird, versprüht werden kann, **dadurch gekennzeichnet, dass** der Duftdiffusor (10) einen Kopf (13) besitzt, der eine Mischung aus einem Trägergas mit einem Duftstrom in einem geeigneten Tank (12) liefert, wobei der genannte Kopf (13) im Austausch mit der Diffusionskammer des diffundierenden Elements steht und über einen Auslass verfügt, durch den der erhaltene Duftstrom abgegeben wird und dann in Richtung des diffundierenden Elements (11) getrieben wird, um dann weiter in den Außenbereich der Anzeigevorrichtung getrieben zu werden.

2. Werbeanzeigevorrichtung gemäß einem der vorhergehenden Ansprüche, **dadurch gekennzeichnet, dass** das diffundierende Element (11) sich aus einem Körper mit einer Diffusionskammer (110) zusammensetzt, die den Duftstrom aufnimmt, wobei die Kammer eine erste Öffnung (111) besitzt, welche sich gegenüber der Öffnung (1a) der Anzeigevorrichtung befindet, eine zweite Öffnung (112), die sich gegenüber dem Innenvolumen der Anzeigevorrichtung befindet, wobei in der Öffnung (112) ein Ventilator (113) angebracht ist, um einen Luftstrom aus dem Innenvolumen der Anzeigevorrichtung in Richtung der Kammer (110) zu treiben, sodass dieser Luftstrom sich mit dem in der Kammer enthaltenen Luftstrom vermischt und die erhaltene Mischung in den Außenbereich der Anzeigevorrichtung getrieben wird.

3. Werbeanzeigevorrichtung gemäß den Ansprüchen 1 und 2 zusammengenommen, **dadurch gekennzeichnet, dass** das diffundierende Element (11) einen Einlass (114) für den Duftstrom in der Kammer beinhaltet, der über ein Leitungsrohr (16) an den Auslass vom Kopf (13) des Duftdiffusors angeschlossen ist.

4. Werbeanzeigevorrichtung gemäß Anspruch 3, **dadurch gekennzeichnet, dass** die erste (111) und die zweite (112) Öffnung der Diffusionskammer (110) des diffundierenden Elements (11) einander gegenüberliegen, und dass der Einlass (114) des Duftstroms in die genannte Kammer (11) schräg oder senkrecht zu einer geometrischen Achse steht, welche die erste (111) und die zweite (112) Öffnung kreuzt, wobei der genannte Einlass auf die erste Öffnung hin ausgerichtet ist.

5. Werbeanzeigevorrichtung gemäß einem der vorhergehenden Ansprüche, **dadurch gekennzeichnet, dass** der Duftstrom ein duftendes Gas ist, weshalb der Duftdiffusor (10) einen Tank mit duftendem Druckgas enthält, welcher über eine Rohrleitung mit dem Mischkopf (13) verbunden ist.

6. Werbeanzeigevorrichtung gemäß einem der vorhergehenden Ansprüche, **dadurch gekennzeichnet, dass** der Duftstrom eine duftende Flüssigkeit ist, weshalb der Duftdiffusor (10) einen Tank mit duftender Flüssigkeit enthält, der mit dem Mischkopf (13) verbunden ist, wobei dieser Kopf auch das Zerteilen der Flüssigkeit in feine Teilchen gewährleistet.

7. Werbeanzeigevorrichtung gemäß Anspruch 6, **dadurch gekennzeichnet, dass** die versprühte Mischung ein Aerosol und der Duftdiffusor (10) ein Vernebler ist.

8. Werbeanzeigevorrichtung gemäß einem der vorhergehenden Ansprüche, **dadurch gekennzeichnet, dass** der Duftdiffusor (10) in zwei separate Module abgetrennt wird, von denen das erste eine Einheit für den Funktionsbefehl und die Funktionssteuerung des Duftdiffusors sowie einen Kompressor, der von einem Elektromotor betrieben wird, enthält, und das zweite den Tank (12) und den Kopf (13) beinhaltet.

9. Werbeanzeigevorrichtung gemäß einem der vorhergehenden Ansprüche, **dadurch gekennzeichnet, dass** der Kopf (13) am Rand des Tanks (12) montiert ist.

10. Werbeanzeigevorrichtung gemäß einem der vorhergehenden Ansprüche, **dadurch gekennzeichnet, dass** der Tank (12) und der Kopf (13) an einer Halterung befestigt ist, die abnehmbar innerhalb eines der Seitenteile (3) des Rahmens (2) der Anzeigevorrichtung (1) fixiert sind.

11. Werbeanzeigevorrichtung gemäß Anspruch 10, **dadurch gekennzeichnet, dass** die Halterung (17) einen horizontalen Stutzen (15) trägt, der dazu dient, sich mit der Buchse (133) des Mischkopfs (13) formschüssig zu verbinden, wobei dieser Stutzen (15) über eine Rohrleitung (14) an den Druckgasauslass eines Kompressors, der sich im Duftdiffusor (10) befindet, angeschlossen ist.

12. Werbeanzeigevorrichtung gemäß Anspruch 10 oder Anspruch 11, **dadurch gekennzeichnet, dass** die Halterung (17) eine vertikale Düse (18) zum Anschluss an die Öffnung des Auslasses (135) des Mischkopfs aufweist, wobei die Düse (18) über eine Rohrleitung (16) mit dem diffundierenden Element (11) verbunden ist.

13. Werbeanzeigevorrichtung gemäß Anspruch 12, **dadurch gekennzeichnet, dass** der Anschlussstutzen (18) von der Halterung (17) schwebend getragen wird und durch ein elastisches Teil (19) gegen die Öffnung des Auslasses (135) des Mischkopfs (13) gehalten wird.

14. Werbeanzeigevorrichtung gemäß Anspruch 13, **dadurch gekennzeichnet, dass** der Anschlussstutzen (18), der über einen zylindrischen Abschnitt seines Körpers durch einen oberen Rand (181) und einen unteren Rand (182) eingegrenzt wird, locker in eine Bohrung eingreift, die in einem horizontalen Flügel (172) der Halterung (17) angelegt ist, wobei die funktionelle Lockerheit des Anschlussstutzens (18) einen Spielraum für eine axiale Verschiebung sowie für eine begrenzte Drehung bietet, und der Mischkopf (13) und der Tank (12) unter dem Flügel (172) positioniert sind.

15. Werbeanzeigevorrichtung gemäß Anspruch 14, **dadurch gekennzeichnet, dass** das elastische Teil (19) eine Spiralfeder ist, die um den zylindrischen Abschnitt des Anschlusstutzenkörpers (18) herum angeordnet ist und zwischen dem horizontalen Flügel (172) der Halterung (17) und dem unteren Rand (182) des Anschlussstutzens (18) eingezwängt ist.

16. Werbeanzeigevorrichtung gemäß einem der Ansprüche 13 bis 15, **gekennzeichnet durch** ein Anschlagsteil (20), das am horizontalen Flügel (172) der Halterung (17) fixiert ist, wodurch ein nach unten gerichteter Ansatz gebildet wird, unter dem die Oberseite des Kopfes (13) anschließt, wobei das Anschlagsteil (20) unter anderem dazu dient, die Drehbewegung um den Stutzen (15) des Kopfes (13) und des Tanks (12) in eine Richtung einzuschränken, die der Winkelbewegung der Öffnung (135) im Verhältnis zur Düse (18) entspricht, und wobei die Düse (18) der Drehbewegung auf der gegenüberliegenden Seite entgegenwirkt.

17. Werbeanzeigevorrichtung gemäß einem der Ansprüche 10 bis 16, **dadurch gekennzeichnet, dass** die Halterung (17) mit einem System (21) für eine abnehmbare Befestigung durch Anhaften im entsprechenden Seitenteil (3) des Rahmens (2) der Tafel (1) ausgerüstet ist, wobei dieses Befestigungssystem mit mindestens einem der seitlichen Flügel dieses Seitenteils zusammenwirkt.

18. Werbeanzeigevorrichtung gemäß Anspruch 17, **dadurch gekennzeichnet, dass** das abnehmbare Befestigungssystem (21) durch Anhaften mindestens einen Klemmzapfen (22) umfasst, der am Ende der Handhabungsvorrichtung (23) befestigt ist.

19. Werbeanzeigevorrichtung gemäß Anspruch 18, **dadurch gekennzeichnet, dass** die Handhabungsvorrichtung mindestens einen elastisch flexiblen Arm (231) umfasst, an dessen distalem Ende ein Klemmzapfen (22) befestigt ist, und dass ein Mechanismus vom Typ Schraube (232) und Mutter (233), dessen Mutter (233) unbeweglich am proximalen Ende des Arms (232) befestigt ist und dessen Schraube (232) vertikal angeordnet ist, in eine Durchgangsbohrung in einem oberen horizontalen Flügel (173) der Halterung einrastet wobei diese Schraube (232) in der Übersetzung blockiert ist und sich frei im Verhältnis zur Halterung drehen kann, und wobei der genannte Arm (231) sich im Verhältnis zur Schraube (232) schräg erstreckt und an einem fixen radialen Anschlagteil (24) ansteht, sodass durch Bewegen der Schraube (232) die schräge Stellung des Arms verändert wird und der genannte Arm sich dreht und auf dem Anschlagteil (24) entlang gleitet, wodurch der Zapfen (22) sich von der Schraube (232) weg oder zu ihr hinbewegt.

20. Werbeanzeigevorrichtung gemäß Anspruch 19, **dadurch gekennzeichnet, dass** der Arm (231) mit seinem distalen Bereich in eine Öffnung (241) eingerastet ist, die sich in einem vertikalen Seitenflügel (171) der Halterung (17) befindet, wobei das fixe radiale Anschlagteil (24) sich aus einem der oberen oder unteren Ränder der Öffnung zusammensetzt, abhängig davon, ob das proximale Ende des Arms (231) höher oder niedriger als das distale Ende ist.

21. Werbeanzeigevorrichtung gemäß Anspruch 20, **dadurch gekennzeichnet, dass** die Handhabungsvorrichtung (23) zwei gegenüberliegende Arme (231) umfasst, von denen jeder einen Klemmzapfen (22) beinhaltet und in zwei Öffnungen (241) eingerastet ist, die in den vertikalen Seitenflügeln (171) der Halterung (17) angebracht sind.

22. Werbeanzeigevorrichtung gemäß Anspruch 21, **dadurch gekennzeichnet, dass** die Handhabungsvorrichtung (23) zwei Paar gegenüberliegende Arme (231) umfasst, nämlich ein Paar obere Arme und ein Paar untere Arme, die mechanisch über einen Verbindungsstab (25), der die Spannung überträgt, miteinander verbunden sind.

23. Werbeanzeigevorrichtung gemäß Anspruch 2, **dadurch gekennzeichnet dass** der Duftdiffusor (10) in einer Diffusionskammer (110) des diffundierenden Elements (11) installiert ist.

24. Werbeanzeigevorrichtung gemäß dem vorhergehenden Anspruch, **dadurch gekennzeichnet, dass** der Duftdiffusor (10) ein Substrat beinhaltet, das mit einem flüchtigen Duftprodukt, welches als Gel oder in flüssiger Form vorliegen kann, durchtränkt ist.

25. Werbeanzeigevorrichtung gemäß Anspruch 23, **dadurch gekennzeichnet, dass** der Duftdiffusor (10) ein Behältnis ist, das Duftprodukte enthält, welche in Form von Kristallen, eines Gels, einer Flüssigkeit oder in irgendeiner anderen Form vorhanden sein können.

26. Werbeanzeigevorrichtung gemäß einem der vorhergehenden Ansprüche, **dadurch gekennzeichnet, dass** der Duftdiffusor (10) und das diffundierende Element (11) sich seitlich des Fensters (7) befinden und durch den peripheren Randbereich (7a) des genannten Fensters verdeckt sind.
